# EUROPEAN PATENT APPLICATION

(11) **EP 4 623 959 A1**
(43) Date of publication of application: **01.10.2025**
(21) Application number: 24165770.9
(22) Date of filing: 25.03.2024
(51) Int. Cl.: A61M 5/31

(54) **STAKED NEEDLE SYRINGE WITH BAKED SILICONE COATING AND METHOD OF MANUFACTURE THEREOF**

(71) Applicant: Becton, Dickinson and Company, Franklin Lakes, NJ 07417-1880 (US)
(72) Inventor: RENZETTI, Philippe, 38920 CROLLES (FR)
(74) Representative: Germain Maureau

(57) **Abstract**

Provided herein is a method for manufacturing a staked needle syringe (10). The method includes providing a glass syringe barrel (12) having a cylindrical main body (40) and a tip (30) positioned at a distal end of the cylindrical main body, applying a silicone-based emulsion onto a least a portion of an inner surface of the glass syringe barrel via a lubricant applicator (70), annealing the silicon lubricant in a baking oven (72), to form a baked silicone coating on the glass syringe barrel and, subsequent to annealing of the silicon lubricant, securing a needle (34) directly into the tip of the glass syringe barrel.

## Description

### BACKGROUND OF THE INVENTION

### Field of the Invention

The present disclosure relates generally to syringes and, more particularly, to staked needle syringes having a baked silicone coating thereon, along with a method of manufacturing such staked needle syringes.

### Description of Related Art

Syringes are used in a variety of environments for administering fluids, such as medications or other drugs, to a patient. A syringe will typically include a glass (or plastic) syringe barrel with an opening at a distal end and a plunger assembly inserted through the opposite proximal end of the barrel. The distal end of the syringe barrel may include a tip through which the opening is formed. In some embodiments, the tip may be configured as a luer connection that enables a transfer of fluid out from or into the syringe barrel, while in other embodiments the tip may be configured as a hub that retains a needle therein for the syringe. The plunger assembly typically includes an elongated plunger rod extending out of the barrel and a plunger head or stopper disposed at the distal end of the plunger rod. The stopper is typically made of elastomeric material and is adapted to ensure the container closure integrity of a syringe when the stopper is inserted into the syringe. The plunger assembly is movable within the syringe barrel to force fluid out from an inner volume of the syringe barrel and/or to aspirate fluid into the inner volume of the syringe barrel - with the stopper sliding through the barrel when the plunger assembly is actuated by a user.

In order to improve sliding of the stopper within the barrel, it is known in the art to lubricate the barrel with a lubricant coating, such as a silicone coating as a primary example. The coating can be deposited on the inner walls of the barrel by spraying, dipping or another suitable application technique. The siliconization of the inner wall of the syringe barrel allows a smooth movement or gliding of the stopper within the chamber, but it has been found that in some cases the silicone coating may interact with components of the drug or medicament contained within the syringe barrel and alter the drug properties. In order to prevent such interaction of the silicone coating with the drug/medicament, it is common to treat the silicone coating applied to the syringe barrel. As an example, for a silicone coating that comprises an emulsion of poly-(dimethylsiloxane), it is common for the coating to be annealed to form so-called "baked silicone", in order to make the coating less reactant with the drug/medicament contained in the syringe barrel. In the manufacture of syringes that include a baked silicone coating thereon, it is desirable to perform the application of the silicone coating and the annealing thereof as part of a clean room washing and packaging process operation, that not only anneals the baked silicone, but also preps the syringe barrel for application of the silicone coating, assembles syringe components on the syringe barrel, and sterilizes the syringe barrel in preparation for pre-filling thereof with a medicament or other fluid. Currently, options are limited regarding the timing and/or performing of particular process steps included in the clean room washing and packaging operation. For example, as regards to the manufacturing of staked needle syringes, it is recognized that the syringe needle cannot be secured to distal tip of the syringe barrel prior to performing of the annealing of the silicone coating, as the annealing step can burn the adhesive used to secure the needle to the syringe barrel, potentially leading to loosening and/or displacement of the needle. Additionally, for existing manufacturing lines that include an oven for annealing of the silicone coating, such manufacturing lines are currently not equipped to provide for placement/assembly of the needle after completion of the annealing step - i.e., placement and securing of the needle directly to the distal tip of a glass syringe barrel. Instead, the only process currently employed for manufacturing a needle syringe with a baked silicone coating on the syringe barrel is for a pre-assembled plastic tip assembly (including a plastic holder and the needle) to be connected to the glass syringe barrel after completion of the annealing step - with it recognized that the addition of such a pre-assembled plastic tip assembly requires an additional manual assembly/connection operation (i.e., a luer connection of the pre-assembled plastic tip assembly is connected to a Luer hub on the syringe barrel).

Accordingly, a need exists in the art for a system and method for manufacturing a staked needle syringe that includes a baked silicone coating on a glass syringe barrel. The system and method would enable the manufacturing of a staked needle syringe that includes a baked silicone coating on the glass syringe barrel, with the needle being placed and secured directly to the glass syringe barrel without use of a plastic tip holder.

### SUMMARY OF THE INVENTION

Provided herein is a method for manufacturing a staked needle syringe. The method includes providing a glass syringe barrel having a cylindrical main body and a tip positioned at a distal end of the cylindrical main body, applying a silicone-based emulsion onto a least a portion of an inner surface of the glass syringe barrel via a lubricant applicator, annealing the silicon lubricant in a baking oven, to form a baked silicone coating on the glass syringe barrel and, subsequent to annealing of the silicon lubricant, securing a needle directly into the tip of the glass syringe barrel.

In certain configurations, the applying of the silicone-based emulsion, the annealing of the silicone-based emulsion, and the securing of the needle are performed in a clean room environment, as part of a clean room washing and packaging process

In certain configurations, the clean room washing and packaging process further comprises placing a rigid needle shield over the needle and onto the glass syringe barrel.

In certain configurations, placing the rigid needle shield over the needle and onto the glass syringe barrel comprises sticking a distal tip of the needle into a compliant jupe of the rigid needle shield and securing the rigid needle shield to the tip.

In certain configurations, the clean room washing and packaging process further comprises performing a sterilization of the staked needle syringe.

In certain configurations, performing the sterilization comprises performing an ethylene oxide (EtO) sterilization or steam sterilization of the staked needle syringe.

In certain configurations, the clean room washing and packaging process comprises washing and drying the glass syringe barrel prior to application of the silicone-based emulsion.

In certain configurations, the clean room washing and packaging process comprises processing syringes along an assembly line at a rate between 200 parts-per-minute and 500 parts-per-minute.

In certain configurations, securing the needle directly into the tip of the glass syringe barrel comprises applying a curable adhesive onto a proximal end of the needle, bonding the proximal end of the needle within the tip via the curable adhesive, and curing the curable adhesive.

In certain configurations, the curable adhesive comprises a UV-curable adhesive or an epoxy-curable adhesive.

In certain configurations, curing the curable adhesive comprises one of baking the curable adhesive in an epoxy-curing oven, when the curable adhesive is the epoxy-curable adhesive, or exposing the curable adhesive to a UV light, when the curable adhesive is the UV-curable adhesive

In certain configurations, applying the silicone-based emulsion onto the inner surface of the glass syringe barrel comprises spraying the silicone-based emulsion via a spray applicator.

Also provided herein is a staked needle syringe comprising a glass syringe barrel having a cylindrical main body and a tip positioned at a distal end of the cylindrical main body, a baked silicone coating applied onto a least a portion of an inner surface of the glass syringe barrel, and a needle retained directly within the tip of the glass syringe barrel.

In certain configurations, the staked needle syringe further includes a curable adhesive that retains the needle within the tip, the curable adhesive comprising an epoxy-curable adhesive or a UV-curable adhesive.

In certain configurations, the staked needle syringe further includes a rigid needle shield positioned over the needle and secured to the glass syringe barrel.

In certain configurations, the baked silicone coating comprises an annealed emulsion of poly-dimethylsiloxane.

### BRIEF DESCRIPTION OF THE DRAWINGS

FIG. 1 is a perspective view of a staked needle syringe, according to an embodiment of the disclosure;
FIG. 2 is an exploded view of the syringe of FIG. 1;
FIG. 3 is a block schematic diagram of a system for manufacturing a staked needle syringe having a baked silicone coating, according to a non-limiting embodiment described herein; and
FIG. 4 is a flowchart of a method for manufacturing a staked needle syringe having a baked silicone coating, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

Aspects and embodiments of the disclosure are directed to staked needle syringes having a baked silicone coating, along with a method of manufacturing such syringes. The method of manufacturing provides for placement and retention of the needle in a glass syringe barrel as part of a clean room washing and packaging process, with the needle being placed after annealing of the basked silicone coating.

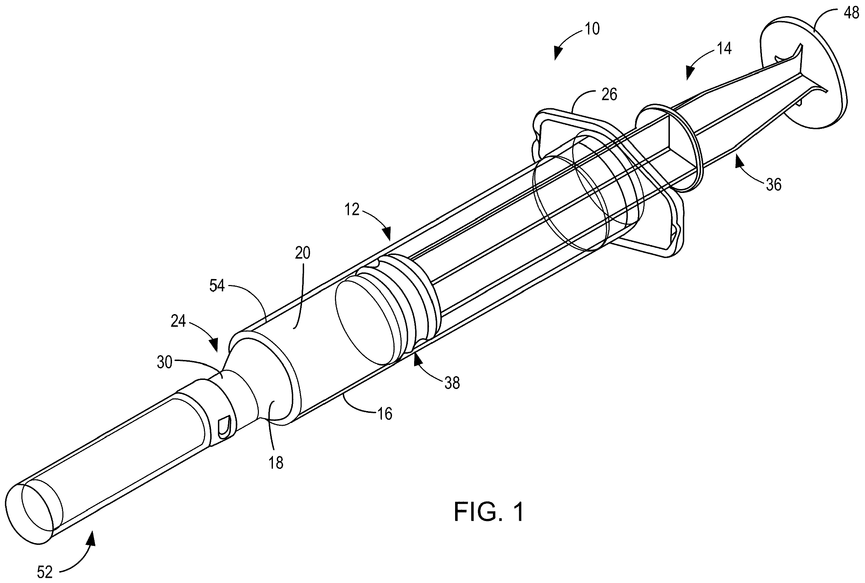

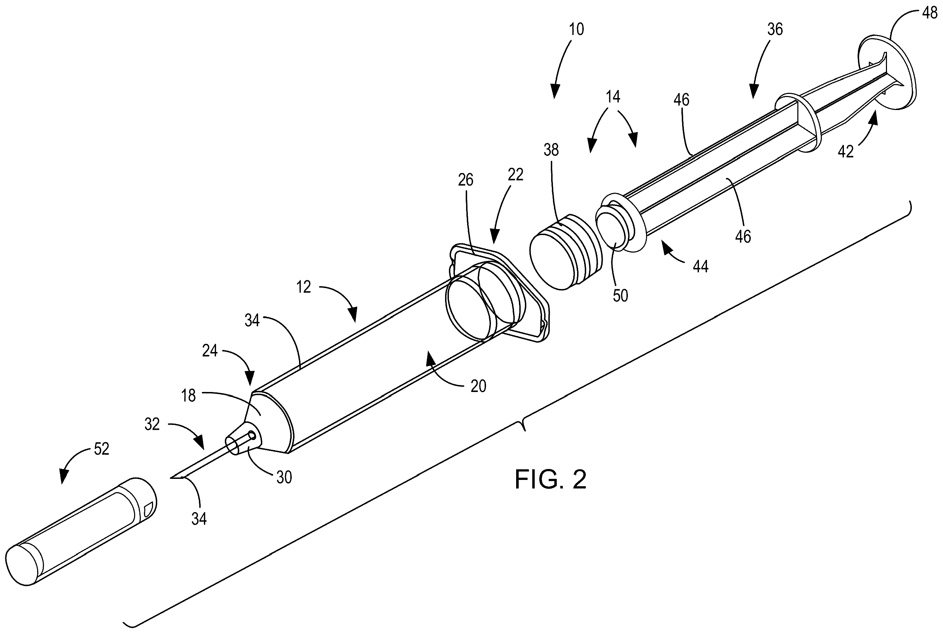

Referring to FIGS. 1 and 2, shown is a non-limiting embodiment of a staked needle syringe with which aspects or embodiments of the disclosure may be implemented.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 includes a tip or hub portion 30 that narrows with respect to the cylindrical outer wall 16 and that extends out distally from end member 18. The hub portion 30 is formed as a partially hollow member that defines a channel 32 therethrough in fluid communication with the chamber 20. A needle 34 is attached to the hub portion 30 within channel 32 (to form a staked needle syringe), with a proximal end of the needle 34 positioned within the channel 32 and a distal end of the needle 34 extending out from the hub portion 30. The proximal end of the needle 34 may be inserted into the channel 32 of hub portion 30 and may be fixed therein with an adhesive (e.g., glue), by thermal welding, or the like. The needle 34 defines a hollow lumen therein that is in fluid communication with the chamber 20 of syringe barrel 12, such that fluid may be dispensed from the syringe 10.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

As shown in FIGS. 1 and 2, a needle shield 52 of syringe assembly 12 is coupled to the syringe barrel 12 to protect the needle 34. According to aspects of the disclosure, the needle shield 52 may be composed of both a rigid material that provides structure to the needle shield 52 and an elastomeric material (i.e., a deformable or compliant material) that surrounds the needle 34 when the needle shield 52 is coupled to the syringe barrel 12.

According to aspects of the disclosure, the syringe barrel 12 may be coated with a lubricant coating 54 in order to improve sliding of the stopper 38 of plunger assembly 14 within the barrel 12. A silicone coating 54, such as an emulsion of poly-(dimethylsiloxane) as a primary example, may be deposited onto the inner walls of the syringe barrel 12 (i.e., on an inner surface of cylindrical wall 16) to allow a smooth movement or gliding of the stopper 38 within the chamber 20. In order to prevent an interaction of the silicone coating 54 with the drug/medicament contained within the syringe barrel 12, the coating 54 may be annealed to form so-called "baked silicone", in order to make the coating less reactant with the drug/medicament contained in the syringe barrel 12.

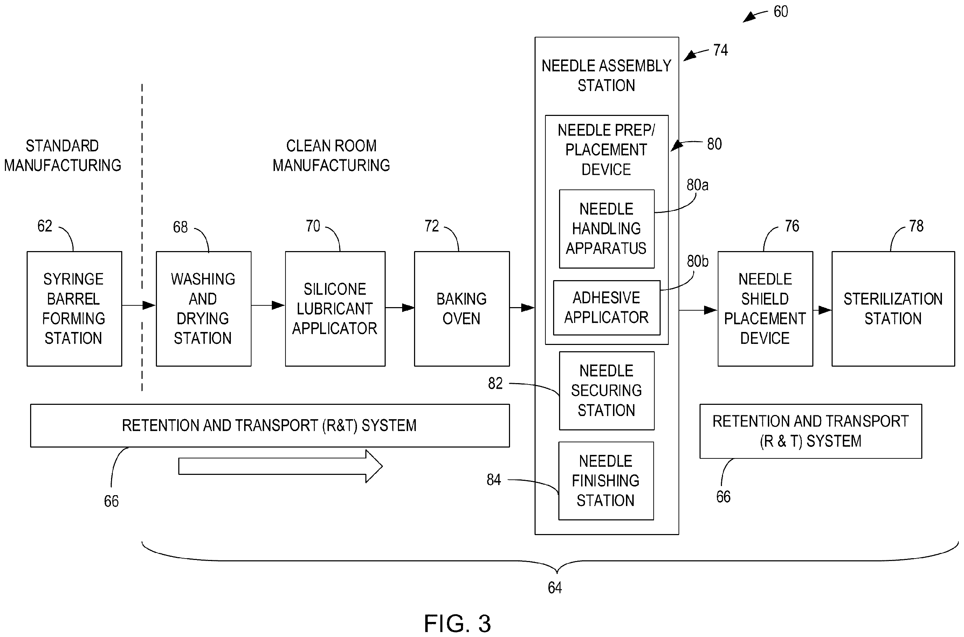

Referring now to FIG. 3, and with continued reference to FIGS. 1 and 2, a block schematic diagram of a system 60 for manufacturing a staked needle syringe (such as syringe 10 shown in FIGS. 1 and 2) having a baked silicone coating is illustrated, according to an embodiment of the disclosure. The system 60 may be characterized as generally including a syringe barrel forming station 62 that operates in a standard manufacturing environment, along with a clean room washing and packaging equipment set-up 64 (hereafter, "clean room equipment 64") that operates in a clean room environment. The clean room equipment 64 may function at any of a number of processing speeds or rates to assemble and otherwise process syringes 10/syringe barrels 12. As non-limiting examples, the clean room equipment 64 may process syringes 10/syringe barrels 12 at a rate of 200 parts-per-minute, 400 parts-per-minute, 450 parts-per-minute, or 500 parts-per-minute, with it recognized that the equipment 64 may be operable at rates/speeds below this range and (potentially) above this range.

The syringe barrel forming station 62 may of a known type that processes blank glass tubes and performs cutting, forming, and annealing steps thereon to output a plurality of syringe barrels 12 configured as previously described with regard to FIGS. 1 and 2. The finished syringe barrels 12 may be loaded onto a retention and transport system 66 for retaining the syringe barrels 12 and advancing the syringe barrels 12 into the clean room environment for processing thereof on the clean room equipment 64. In some embodiments, the retention and transport system 66 may comprise an array of syringe barrel holding pucks (not shown) arranged into a plurality of rows, with holding pucks affixed to a conveyor (not shown) that provides for movement thereof.

As shown in FIG. 3, the clean room equipment 64 included in system 60 may include a washing and drying station 68, a lubricant applicator 70, a baking oven 72, a needle assembly station 74, a needle shield placement device 76, and a sterilization station 78.

In operation of the system 60 and the clean room equipment 64 thereof, the formed syringe barrels 12 are initially provided to the washing and drying station 68 by retention and transport system 66. The washing and drying station 68 may spray the syringe barrels 12 with a fluid that cleans internal and external surfaces of the syringe barrels 12, and may then subsequently dry the syringe barrels 12 after such cleaning. The cleaned and dried syringe barrels 12 are thus prepped for further processing by the clean room equipment 64.

The cleaned and dried syringe barrels 12 may be advanced by retention and transport system 66 to the lubricant applicator 70, which operates to apply a silicone-based emulsion coating (e.g., poly-(dimethylsiloxane)) to the inner surface of the syringe barrels 12. In some embodiments, the lubricant applicator 70 may be configured as a sprayer that emits a silicone emulsion spray onto inner surface of the syringe barrels 12, to provide a thin layer of silicone-based emulsion thereon.

Upon application of the silicone-based emulsion coating to the inner surface of the syringe barrels 12, the retention and transport system 66 delivers the syringe barrels 12 to the baking oven 72. The baking oven 72 functions to bake the silicone-based emulsion coating that has been applied to the syringe barrels 12, such as at a temperature between 150-280°C, in order to anneal the silicone-based emulsion coating and form a baked silicone coating on the inner surface of the syringe barrels 12. Formation of the basked silicone coating provides a coating that is less reactant with a drug/medicament contained in the syringe barrel 12.

According to aspects of the invention, a needle assembly station 74 is included in the clean room equipment 64 in order to enable placement and securing of a staked needle 34 to the syringe barrel 12 subsequent to formation of the baked silicone coating. In some embodiments, the needle assembly station 74 may be comprised of a needle preparation and placement device 80, a needle securing station 82, and a needle finishing station 84.

The needle preparation and placement device 80 may comprise a needle handling apparatus 80a and adhesive applicator 80b. The adhesive applicator 80b may apply an adhesive, such as a ultraviolet (UV) curable adhesive or an epoxy-curable adhesive, onto a proximal end of the needle 34, while the needle handling apparatus 80a may grasp a distal end of the needle 34 and transfer the needle 34 into place in the syringe barrel 12 - i.e., insert the proximal end of the needle 34 (with adhesive applied thereto) into the channel 32 of hub portion 30.

The needle securing station 82 may comprise a device that functions to cure or otherwise set the adhesive applied to the proximal end of the needle 34, such that the proximal end of the needle 34 may be secured within the channel 32 of hub portion 30. In some embodiments, where the adhesive is an epoxy-curable adhesive, the needle securing station 82 may thus comprise an oven that cures the epoxy-curable adhesive applied onto needle 34. In other embodiments, where the adhesive is a UV-curable adhesive, the needle securing station 82 may thus comprise a UV light source that emits UV light that cures the UV curable adhesive applied onto needle 34.

After curing of the curable adhesive and securing of the needle to syringe barrel 12, the needle finishing station 84 may perform any necessary processing steps on the assembled syringe. In some embodiments, the needle finishing station 84 may thus function to: inspect an overall length (OAL) of the needle assembly (i.e., length of the syringe barrel assembled with the needle), inspect a quality of the needle to ensure no needle point damage has occurred, and/or inspect whether the lumen/channel within the needle has been clogged or blocked. It is recognized that other quality control steps/processes may also be performed, beyond those specifically listed herein.

After the needle assembly quality inspections have been completed, a needle siliconization may be performed - with silicon oil being dispended onto the outside of the needle surface, in order to reduce the penetration force of the needle when used for injection.

Following processing of the syringe at the needle assembly station 74, the retention and transport system 66 may deliver the syringe 10 to the needle shield placement device 76 of system 60. The needle shield placement device 76 may apply a rigid needle shield 52 over the needle 34 and onto the distal end of the syringe barrel 12, to seal and provide protection to the needle 34. In some embodiments, in placing the rigid needle shield 52 over the needle 34 and onto the distal end of the syringe barrel 12, the distal end of the needle 34 may be inserted into a compliant jupe of the needle shield 52 (to seal the tip of needle 34), while an open end of the rigid needle shield 52 may mate with the hub portion 30 of syringe barrel 12, in order to secure the needle shield 52 in place.

With the needle shield 52 placed onto the needle 34 and syringe barrel 12, a final processing step of sterilizing the assembled syringe 10 may be performed by the sterilization station 78. In some embodiments, the sterilization performed by the sterilization station 78 may comprise application of a sterilization gas to the syringe 10 - with application of ethylene oxide (EtO) gas or vaporized hydrogen peroxide being non-limiting examples. In other embodiments, the sterilization performed by the sterilization station 78 may comprise exposure of the syringe 10 to radiation (e.g., gamma rays) or to steam ("steam sterilization"), alone or in combination with application of a sterilization gas.

Accordingly, the system 60 operates to manufacture a staked needle syringe 10 having a baked silicone coating thereon. The system 60 incorporates a needle assembly station 74 as part of the clean room equipment 64, with the needle assembly station 74 providing for placement and securing of the needle 34 to the syringe barrel 12 after annealing of the silicone emulsion onto the syringe barrel 12.

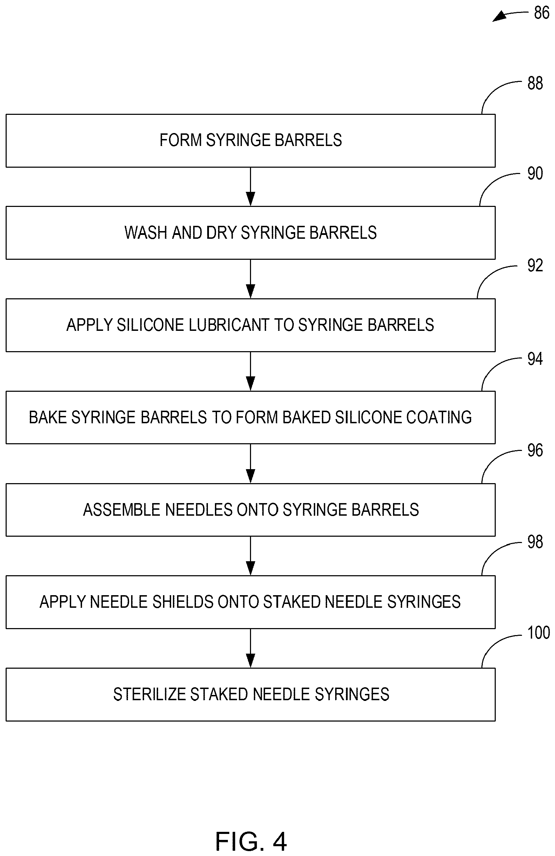

Referring now to FIG. 4, and with continued reference to FIGS. 1-3, a flowchart illustrating a method 86 for manufacturing a staked needle syringe 10 having a baked silicone coating 54 is provided, according to an embodiment of the disclosure.

The method 86 begins at step 88 with the forming of a syringe barrel 12 at a syringe barrel forming station 62. The syringe barrel forming station 62 may be configured to process blank glass tubes and perform cutting, forming, and annealing steps thereon to output a plurality of syringe barrels 12 configured as previously described with regard to FIGS. 1 and 2.

The finished syringe barrels 12 may be advanced into a clean room environment for processing thereof as part of a clean room washing and packaging operation, encompassing steps 90-100, as described hereafter. Initially, the finished syringe barrels 12 undergo a washing and drying process at step 90. A washing and drying station 68 may operate to spray the syringe barrels 12 with a cleaning fluid, and may then subsequently dry the syringe barrels 12 after such cleaning. The cleaned and dried syringe barrels 12 are thus prepped for further processing by clean room equipment 64.

After washing and drying of the syringe barrels 12, the method 86 continues as step 92, where a silicone-based emulsion coating 54 is applied to the inner surface of the syringe barrels 12. In some embodiments, a spray applicator may apply a silicone emulsion spray (e.g., poly-(dimethylsiloxane)) to the inner surface of the syringe barrels 12, to provide a thin layer of silicone-based emulsion thereon.

Upon application of a silicone-based emulsion 54 onto the syringe barrel 12, the syringe barrels 12 are baked within a baking oven 72 at step 94, such as at a temperature between 150-280°C,. The baking oven 72 operates to bake the silicone-based emulsion coating 54 that has been applied to the syringe barrels 12, in order to anneal the silicone-based emulsion coating and form a baked silicone coating on the inner surface of the syringe barrels 12.

After annealing of the silicone-based emulsion coating 54 to form a baked silicone coating, the method 86 continues as step 96, where a needle 34 is assembled with the syringe barrel 12 to form a staked needle syringe. In assembling the needle 34 with the syringe barrel 12, a needle assembly station 74 may perform a series of processing steps. First, the needle 34 may be placed directly into the glass tip/hub portion 30 of the syringe barrel 12 by a needle preparation and placement device 80. In placing the needle into hub portion 30, a proximal end of the needle 34 - having a curable adhesive applied thereto, such as an epoxy-curable adhesive or UV-curable adhesive - is placed into the channel 32 of hub portion 30. Upon placement of the needle 34 into hub portion 30, the adhesive may be cured, such as by passing the staked needle syringe 10 through an oven (if an epoxy-curable adhesive) or exposing the staked needle syringe 10 to UV light (if a UV-curable adhesive), in order to secure the needle 34 to syringe barrel 12. Step 96 may also optionally include additional processing steps, including needle inspection (OAL inspection, needle tip quality/damage inspection, and/or needle clogging inspection) and/or siliconization of an outer surface of the needle.

Following processing of the syringe 10 at the needle assembly station 74, a rigid needle shield 52 may be applied over the needle 34 and onto the distal end of the syringe barrel 12 at step 98, so as to seal and provide protection to the needle 34. In some embodiments, in placing the rigid needle shield 52 over the needle 34 and onto the distal end of the syringe barrel 12, the distal end of the needle 34 may be inserted into a compliant jupe of the needle shield 52 (to seal the tip of needle 34), while an open end of the rigid needle shield 52 may mate with the hub portion 30 of syringe barrel 12, in order to secure the needle shield 52 in place.

With the needle shield 52 placed onto the needle 34 and syringe barrel 12, the method 86 continues at step 100, where the staked needle syringe 10 is sterilized. In some embodiments, the sterilization may comprise application of a sterilization gas to the syringe 10 - with application of ethylene oxide (EtO) gas or vaporized hydrogen peroxide being non-limiting examples. In other embodiments, the sterilization may comprise exposure of the syringe 10 to radiation (e.g., gamma rays) or steam ("steam sterilization"), alone or in combination with application of a sterilization gas.

Accordingly, the method 86 provides for manufacturing of a staked needle syringe 10 having a baked silicone coating 54 thereon. The method 86 provides for placement and retention of a needle 34 directly in the tip 30 of a glass syringe barrel 12, as part of a high-speed SCF process performed in a clean room environment, with the needle 34 being placed/retained in the syringe barrel 12 after annealing of the silicone emulsion coating 54 onto the syringe barrel 12 - i.e., after formation of the baked silicone layer.

Beneficially, embodiments of the invention thus provide a system and method for manufacturing staked needle syringes that includes a baked silicone coating on a glass syringe barrel. The system and method enable the manufacturing of staked needle syringes that include a baked silicone coating on the glass syringe barrel, with the needle being placed and secured directly to the glass syringe barrel without use of a plastic tip holder. Needle assembly equipment and a baking oven (for annealing silicone-based emulsion coating) are integrated into a clean room washing and packaging process, in order to produce the staked needle syringes.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A method for manufacturing a staked needle syringe (10), the method comprising:
providing a glass syringe barrel (12) having a cylindrical main body (40) and a tip (30) positioned at a distal end of the cylindrical main body;
applying a silicone-based emulsion onto a least a portion of an inner surface of the glass syringe barrel via a lubricant applicator (70);
annealing the silicon lubricant in a baking oven (72), to form a baked silicone coating on the glass syringe barrel; and
subsequent to annealing of the silicon lubricant, securing a needle (34) directly into the tip of the glass syringe barrel.

2. The method of claim 1, wherein the applying of the silicone-based emulsion, the annealing of the silicone-based emulsion, and the securing of the needle (34) are performed in a clean room environment, as part of a clean room washing and packaging process.

3. The method of claim 2, wherein the clean room washing and packaging process further comprises placing a rigid needle shield (52) over the needle (34) and onto the glass syringe barrel (12); and
wherein, optionally, placing the rigid needle shield over the needle and onto the glass syringe barrel comprises sticking a distal tip of the needle into a compliant jupe of the rigid needle shield and securing the rigid needle shield to the tip (30).

4. The method of any of claims 2-3, wherein the clean room washing and packaging process further comprises performing a sterilization of the staked needle syringe (10).

5. The method of claim 4, wherein performing the sterilization comprises performing an ethylene oxide (EtO) sterilization or steam sterilization of the staked needle syringe (10).

6. The method of any of claims 2-5, wherein the clean room washing and packaging process comprises washing and drying the glass syringe barrel (12) prior to application of the silicone-based emulsion.

7. The method of any of claims 2-6, wherein the clean room washing and packaging process comprises processing syringes along an assembly line at a rate between 200 parts-per-minute and 500 parts-per-minute.

8. The method of any of claims 1-7, wherein securing the needle (34) directly into the tip (30) of the glass syringe barrel (12) comprises:
applying a curable adhesive onto a proximal end of the needle;
bonding the proximal end of the needle within the tip via the curable adhesive; and
curing the curable adhesive.

9. The method of claim 8, wherein the curable adhesive comprises a UV-curable adhesive or an epoxy-curable adhesive.

10. The method of claim 9, wherein curing the curable adhesive comprises one of:
baking the curable adhesive in an epoxy-curing oven when the curable adhesive is the epoxy-curable adhesive; or
exposing the curable adhesive to a UV light when the curable adhesive is the UV-curable adhesive.

11. The method of any of claims 1-10, wherein applying the silicone-based emulsion onto the inner surface of the glass syringe barrel (12) comprises spraying the silicone-based emulsion via a spray applicator.

12. A staked needle syringe (10) comprising:
a glass syringe barrel (12) having a cylindrical main body (40) and a tip (30) positioned at a distal end of the cylindrical main body;
a baked silicone coating applied onto a least a portion of an inner surface of the glass syringe barrel; and
a needle (34) retained directly within the tip of the glass syringe barrel.

13. The staked needle syringe (10) of claim 12, further comprising a curable adhesive that retains the needle (34) within the tip (30), the curable adhesive comprising one of a UV-curable adhesive or an epoxy-curable adhesive.

14. The staked needle syringe (10) of any of claims 12-13, further comprising a rigid needle shield (52) positioned over the needle (34) and secured to the glass syringe barrel (12).

15. The staked needle syringe (10) of any of claims 12-14, wherein the baked silicone coating comprises an annealed emulsion of poly-dimethylsiloxane.
